(19) Europäisches Patentamt
European Patent Office
Office européen des brevets

(11) Publication number: **0 287 751 B1**

(12) **EUROPEAN PATENT SPECIFICATION**

(45) Date of publication of patent specification: **28.12.94**

(51) Int. Cl.5: **C07D 501/22**, A61K 31/545

(21) Application number: **88100478.2**

(22) Date of filing: **14.01.88**

# (54) Crystalline cefadroxil and method for producing it.

(30) Priority: **24.04.87 GB 8709804**
**04.08.87 GB 8718394**

(43) Date of publication of application:
**26.10.88 Bulletin 88/43**

(45) Publication of the grant of the patent:
**28.12.94 Bulletin 94/52**

(84) Designated Contracting States:
**AT BE CH DE ES FR GB GR IT LI LU NL SE**

(56) References cited:
**FR-A- 2 349 589**
**FR-A- 2 365 570**

(73) Proprietor: **RIFAR S.R.L.**
**Via Borromei, 9**
**I-20123 Milan (IT)**

(72) Inventor: **Marsili, Leonardo**
**Residenza del Cantone 731**
**Milano 2**
**I-20090 Segrate (IT)**

(74) Representative: **Giambrocono, Alfonso, Dr. Ing. et al**
**Ing. A. Giambrocono & C. S.r.l.**
**Via Rosolino Pilo 19/B**
**I-20129 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to a method for producing a novel crystalline cefadroxil.

Cefadroxil is a well known antibiotic substance having antibacterial activity: it is disclosed and claimed in the U.S. patent no. 3,489,752 according to which it is obtained by acylation of 7-ADCA with an amino-protected derivative of D(-)-alpha-p-hydroxyphenylglycine. U.S. patent no. 3,985,741 discloses preparation of cefadroxil by acylation of 7-ADCA with mixed anhydride of D-(-)-alpha-p-hydroxyphenylglycine when the latter's alpha-amino group has been blocked with a beta-keto compound such as methyl acetoacetate: the reaction mixture is first added with water and then with dimethylformamide to precipitate a crystallized solvate of cefadroxil having more than 3% of water content which, after filtration, is slurried in 90% methanol.

U.S. patent no. 4,504,657 describes and claims a different form of cefadroxil, which is the crystalline cefadroxil monohydrate having a well defined X-ray diffraction pattern characterizing said compound: this crystalline cefadroxil monohydrate is obtained (see also the U.S. patent RE 31,730) by acylation of silylated 7-ADCA with D(-)-alpha-p-hydroxyphenylglycine chloride hydrochloride. The reaction mixture is added first with water and then with dimethylformamide to precipitate the dimethylformamide solvate which after filtration is treated with water or a water/solvent mixture to cleave the solvate and to precipitate the desired final compound.

The present invention relates to a method for the production of a novel crystalline cefadroxil having a water content of about 3% and characterized by the following X-ray diffraction properties:

| Spacing d(Å) | Relative Intensity |
|---|---|
| 10.59 | 30 |
| 8.68 | 100 |
| 8.03 | 43 |
| 7.14 | 39 |
| 6.72 | 41 |
| 6.18 | 40 |
| 5.67 | 11 |
| 5.49 | 23 |

| | |
|---|---|
| 4.83 | 69 |
| 4.71 | 52 |
| 4.65 | 25 |
| 4.23 | 57 |
| 4.13 | 55 |
| 3.98 | 74 |
| 3.87 | 33 |
| 3.80 | 36 |
| 3.31 | 30 |
| 3.02 | 15 |
| 2.95 | 18 |
| 2.88 | 25 |
| 2.64 | 25 |
| 2.55 | 19 |
| 2.52 | 17 |
| 2.48 | 14 |
| 2.43 | 15 |
| 2.31 | 16 |
| 2.14 | 13 |
| 2.04 | 9 |

This novel cefadroxil in the following will be called "cefadroxil hemihydrate".

The cefadroxil hemihydrate is obtained by adding to an aqueous solution containing cefadroxil just prepared from 7-ADCA a solvent selected from the group consisting of dimethylacetamide, N-methyl-2-pyrrolidone, monomethylformamide, while controlling the pH of the solution in the range 5.5-6, to give the corresponding cefadroxil solvate which precipitates and is filtered off.

After drying, the filtered precipitate is slurried with a mixture methanol-isopropyl alcohol in the ratio of 30:70 to 70:30 by volume containing from 5% to 12% of water, at a temperature in the range of +45 °C to -55 °C and than isolating the so obtained crystalline cefadroxil hemihydrate.

All known methods for transforming the 7-ADCA into the mentioned aqueous solution containing cefadroxil can be used. For instance, it is possible to follow the procedures described in Example XVI of the European patent application no. 001,133, or in Example 1 and 2 of the U.K. patent application no. 2,064,511 or in Examples 1 to 4 of the U.S. patent no. 4,234,721.

Attempts have been made to use also the known cefadroxil-dimethylformamide solvate (see the U.S. patents nos. 3,985,741 4,504,657 and Re 31,730) but it was impossible to obtain the desired final cefadroxil hemihydrate (always the known crystalline cefadroxil monohydrate was isolated): this appears to be due to the fact that the cefadroxil -DMF solvate has a K.F. value of 1.8% or more, as it is written also in Example 6A of U.S. patent no. 3,985,741 and in Example 2A of the U.S. patent no. 4,234,721.

The use of the cefadroxil solvates of dimethylacetamide, of N-methyl-2-pyrrolidone and of monomethylformamide is therefore critical, what may be due to their very low water content (<1.0%); such solvates are novel and essential intermediates for the production of the cefadroxil hemihydrate and therefore they are part of the present invention as obtained by the here claimed method.

Also the use of the mixture methanol/isopropyl alcohol has proved to be essential: indeed, if ethyl alcohol is used, the cefadroxil molecule is decomposed; if methanol alone is used, the molecule of the final cefadroxil retains an exceedingly high amount of the same methanol (more than 0.4%), what is unacceptable in consequence of its toxicity. This has proved to be true also in the case that methanol is used in mixture with other alcohols wherein the methanol content is too high.

The isopropyl alcohol does not decompose the cefadroxil molecule and its toxicity is much lower than that of methanol, but it has been found that it cannot be used alone because it is impossible to obtain pure crystalline cefadroxil hemihydrate.

It has been surprisingly found that if isopropyl alcohol is additioned with methanol in an amount of 30 to 70% of isopropyl alcohol and 70 to 30% of methanol, the novel crystalline cefadroxil hemihydrate with a methanol content lower than 0.010% (corresponding to 100 ppm) is obtained.

The invention is illustrated by the following examples in which the NMR spectra were recorded in $D_2O$ solution (15mg/ml) on a Varian XL-300 spectrometer.

<u>EXAMPLE 1</u> Cefadroxil Dimethylacetamide Solvate.

7-ADCA (45g) was added to methylene chloride (700 ml) at room temperature. Triethylamine (35.5 g) was added over 15′ with stirring at a temperature below 25 °C. Trimethylchlorosilane (43.2 g) was then dropped over a 30′ period. The mixture was stirred at 30 °C for 90′ and then cooled to -10 °C.

Dimethylaniline (31 g) and D(-)-p-hydroxyphenylglycyl chloride hydrochloride hemidioxane solvate (63g) were added and the mixture was stirred at -5 °C/0 °C for about 90′. Water (170 ml) was added and the reaction mixture was stirred for 30′. The aqueous phase was diluted with dimethylacetamide (350 ml) and the pH was adjusted to 6.0 by slowly adding diethylamine at 25 °C. The mixture was stirred at 20 °C for 120′. The cefadroxil dimethylacetamide solvate was collected by filtration, washed with dimethylacetamide/water 2:1 then with acetone to yield, after drying at 40 °C, 81.3 g of the title compound
K.F.: 0.51%
HPLC Assay: 69.3% on dry basis
PMR: 6.9 - 7.35 δ (m,$C_6H_4$-); 5.59 δ [d, C(7)-H]; 5.15 δ (s,CH-CO); 4.98 δ [d, CH-S];3.02-3.42 δ (m, S-$CH_2$); 1.8 δ (s, $CH_3$) characteristic of cefadroxil moiety and the following peaks due to the solvent:
2.83-3.01 δ (s,s $N(CH_3)_2$); 2.04 δ (d,$COCH_3$)
$^{13}$C-NMR: 21.07 δ [$CH_3$-C=]; 30.93 δ [$CH_2$-S]; 58.78 δ [CH-$NH_2$]; 59.51 δ [CH-S]; 61.16 δ [NH-CH-CO]; 124.60 δ

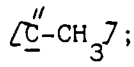

126.11 δ

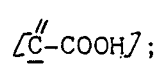

166.21 δ [CO, β-lactam]; 172.37 δ [COOH]; 172.58 δ [CO-NH];129.05 δ, 132.7 δ, 118.99 δ, 160.45 δ [aromatic carbon atoms] characteristic of Cefadroxil moiety and the following peaks due to the solvent:
23.15 δ [CO-$CH_3$]; 37.93 δ [N-$CH_3$]; 40.85 δ [N-$CH_3$]; 176.74 δ [CO].

EXAMPLE 2 Cristalline Cefadroxil hemihydrate

Cefadroxil dimethylacetamide solvate (50g) prepared according to Example 1 was slurried in a mixture of isopropyl alcohol (250 ml) and methanol (120 ml) in the presence of 24 ml of water at 48 °C-50 °C.

After 120′ the mixture was cooled to 10 °C, filtered and washed with acetone to yield 34.5g of crystalline Cefadroxil hemihydrate.

K.F.: 2.8 %

Methanol: 0.009 %

Isopropyl alcohol: 0.17 %

HPLC assay: 99.1 % on dry basis

The powder exhibits the following X-ray diffraction properties determined with the radiation (wave lenght: Lambda 1.54051 Angstrom) produced with a Cu/Ni X-ray tube:

| Spacing d(Å) | Relative Intensity |
|---|---|
| 10.59 | 30 |
| 8.68 | 100 |
| 8.03 | 43 |

| | |
|---|---|
| 7.14 | 39 |
| 6.72 | 41 |
| 6.18 | 40 |
| 5.67 | 11 |
| 5.49 | 23 |
| 4.83 | 69 |
| 4.71 | 52 |
| 4.65 | 25 |
| 4.23 | 57 |
| 4.13 | 55 |
| 3.98 | 74 |
| 3.87 | 33 |
| 3.80 | 36 |
| 3.31 | 30 |
| 3.02 | 15 |
| 2.95 | 18 |
| 2.88 | 25 |
| 2.64 | 25 |
| 2.55 | 19 |
| 2.52 | 17 |

| | |
|---|---|
| 2.48 | 14 |
| 2.43 | 15 |
| 2.31 | 16 |
| 2.14 | 13 |
| 2.04 | 9 |

EXAMPLE 3: Cefadroxil Monomethylformamide Solvate

7-ADCA (30g) was added to methylene chloride (450 ml), trimethylchlorosilane (28.8 g) was added and the mixture was stirred for 10′. Triethylamine (23.7 g) was then dropped over a 30′ period while temperature was allowed to reach 30 ° C. The mixture was stirred 2 hours at 30 ° C and then cooled to -10 ° C.

Bis-trimethyl-silyl-urea (21 g) and D(-)-p-hydroxyphenylglycyl chloride hydrochloride hemidioxane solvate(45g) were added and the mixture was allowed to react at -5 ° C for 90′. After additional 30′ stirring at 0 ° C, water (115 ml) was added.

The reaction mixture was stirred for 30′, the aqueous layer cooled to 5 ° C and diluted with monomethylformamide (240 ml). Triethylamine was added slowly over 60′ and the pH was adjusted to 5.7 at 20 ° C. After stirring for 2 hours the slurry was filtered, the filter cake washed with monomethylformamide/water 2:1 and then with acetone to yield, after drying at 40 ° C, 49 g of the title compound:
K.F.: 0.9 %
HPLC Assay: 79.8% on dry basis
PMR: besides the peaks characteristic of Cefadroxil moiety shown in example 1, the following peaks are due to the solvent 7.98 δ [s, HCO]; 2.71 δ cis [s, $NHCH_3$]
$^{13}C$-NMR: besides the peaks characteristic of Cefadroxil moiety shown in Example 1, the following peaks are due to the solvent:
27.07 δ [$\underline{C}H_3$]; 167.6 δ [H-$\underline{C}$O].

EXAMPLE 4: Crystalline Cefadroxil hemihydrate

Cefadroxil monomethylformamide solvate (30g) prepared according to Example 3 was slurried in 150 ml of a mixture 1:1 of methanol and isopropyl alcohol in the presence of 16 ml of water at 52 ° C. After 70′ at 52 ° C the mixture was cooled to 10 ° C, filtered and washed with acetone to yield 23.2g of crystalline cefadroxil hemihydrate.
K.F.: 2.9%
HPLC Assay: 99.6% on dry basis
Methanol: 0.008%
Isopropyl alcohol: 0.15%
The powder exhibits the same X-ray diffraction properties of the product obtained in Example 2.

EXAMPLE 5 Cefadroxil Dimethylacetamide Solvate

Potassium methyl Dane salt of D(-)-p-hydroxy-phenylglycine (30.3 g) was added to acetone (170 ml) and the mixture was cooled to -40 ° C.

Ethylchlorocarbonate (11.15 g) and N-methylmorpholine (0.25 ml) were added at -40 ° C. The temperature was kept at -35 ° C for 120′ and then the mixture was cooled to -55 ° C.

7-ADCA (21.5g) was charged at +5 ° C into water (50 ml) and dimethylsuphoxide (90 ml) and triethylamine (11.3 g) were added. The obtained solution was cooled to 0 ° C and the suspension of mixed anhydride (at -55 ° C) was added to the solution of 7-ADCA.

The mixture was stirred at -25 ° C for 60′; the temperature was raised to 0 ° C and HCl 37% was added slowly during 60′ to a constant pH 1.8. Methylene chloride (175 ml), was added and the mixture was stirred for 15′. The upper layer was diluted with dimethylacetamide (170 ml) and acetone (70 ml), the pH was

adjusted to 6.5 at 0°C with triethylamine. The mixture was stirred at 0°C for 2 hours. The solvate was washed with dimethylacetamide/water 2:1 and then with acetone to yield 40.5 g of the title compound after drying at 40°C.
K.F.: 0.63%
HPLC Assay: 69.1% on dry basis

EXAMPLE 6 Cefadroxil 1-Methyl-2-pyrrolidone Solvate

7-ADCA (30 g) was reacted according to the procedure described in Example 1 using 1-methyl-2-pyrrolidone instead of dimethylacemide. Yield: 52g
K.F.: 0.85%
HPLC Assay: 68.7% on dry basis
PMR: besides the peaks characteristic of Cefadroxil moiety shown in Example 1, the following peaks are due to the solvent 3.45 δ [t, $CH_2$(5)]; 2.36 δ [t, $CH_2$(3)]; 1.98 δ [q, $CH_2$(4)]; 2.84 δ [s, N-$CH_3$)].
$^{13}$C-NMR: besides the peaks characteristic of Cefadroxil moiety shown in example 1, the following peaks are due to the solvent:
19.71 δ [$\underline{C}H_2$(4)]; 32.27 δ [N, $\underline{C}H_3$]; 33.45 δ [$\underline{C}H_2$(3)]; 52.97 δ [$\underline{C}H_2$(5)]; 180.84 δ [$\underline{C}$O (2)].

EXAMPLE 7 Crystalline Cefadroxil hemihydrate

Cefadroxil 1-methyl-2-pyrrolidone solvate (30g) was slurried in a mixture of 100 ml isopropyl alcohol and 50 ml of methanol kept at 45-48°C in the presence of 19 ml of water for 100′. After cooling to 10°C the mixture was filtered, the produce washed with acetone and dried at 40°C.
Yield: 19.5 g of hemihydrate product
K.F.: 2.5%
HPLC Assay: 98.2 % on dry basis
Methanol: 0.009%
Isopropyl alcohol: 0.18%

EXAMPLE 8 Crystalline Cefadroxil Hemidrate

Cefadroxil dimethylacetamide solvate (40 g) was slurried in a mixture of isopropyl alcohol (100 ml), methanol (200 ml) and water (30 ml) at 18°-50°C.
After 120′ the mixture was cooled to 10°C, filtered and washed with acetone to yield 26.8 g of crystalline cefadroxil hemihydrate.
K.F. 3.1%
HPLC Assay: 98.7% on dry basis
Methanol: 0.008%
Isopropyl alcohol: 0.02%

EXAMPLE 9 Crystalline Cefadroxil Hemidrate

Cefadroxil monomethylformamide solvate (30 g) was slurried in a mixture of methanol (100 ml), isopropyl alcohol (47 ml) and water (13 ml) at 50°C. After 90′ at 50°C the mixture was cooled to 10°C, filtered and washed with acetone to yield 22.9 g of crystalline cefadroxil hemihydrate.
K.F. 3.0%
HPLC Assay: 99.1% on dry basis
Methanol: 0.008%
Isopropyl alcohol: 0.010%

EXAMPLE 10 Crystalline Cefadroxil hemidrate

Cefadroxil 1-methyl-2-pyrrolidone solvate (30 g) was slurried in a mixture of metahnol (80 ml), isopropyl alcohol (60 ml) and water (10 ml) at 50°C for 90′. After cooling to 10°C the mixture was filtered, the product washed with acetone and dried at 40°C to yield 19.1 g of crystalline cefadroxil hemihydrate.
K.F.: 2.9%
HPLC Assay: 98.9 on dry basis.
Methanol: 0.009%

Isopropyl alcohol: 0.16%

## Claims

**1.** A method for the production of crystalline cefadroxyl hemihydrate exhibiting essentially the following X-ray diffraction properties:

| Spacing d(Å) | Relative Intensity |
|---|---|
| 10.59 | 30 |
| 8.68 | 100 |
| 8.03 | 43 |
| 7.14 | 39 |
| 6.72 | 41 |
| 6.18 | 40 |
| 5.67 | 11 |
| 5.49 | 23 |
| 4.83 | 69 |
| 4.71 | 52 |
| 4.65 | 25 |
| 4.23 | 57 |
| 4.13 | 55 |
| 3.98 | 74 |
| 3.87 | 33 |

| | |
|---|---|
| 3.80 | 36 |
| 3.31 | 30 |
| 3.02 | 15 |
| 2.95 | 18 |
| 2.88 | 25 |
| 2.64 | 25 |
| 2.55 | 19 |
| 2.52 | 17 |
| 2.48 | 14 |
| 2.43 | 15 |
| 2.31 | 16 |
| 2.14 | 13 |
| 2.04 | 9 |

wherein to an aqueous solution of cefadroxil just prepared from 7-ADCA there is added a solvent selected from the group consisting of dimethylacetamide, N-methyl-2-pyrrolidone, monomethylformamide, while controlling the pH of the solution in the range 5.5-6, to give the corresponding cefadroxil solvate which precipitates and is filtered off and which after drying is slurried with a mixture methanol:isopropyl alcohol 30:70 to 70:30 by volume containing from 5% to 12% of water, at a temperature in the range of +45°C to +55°C the so obtained crystalline cefadroxil hemihydrate being then isolated from the reaction mixture.

**2.** Cefadroxil solvates of dimethylacetamide, of N-methyl-2-pyrrolidone and of monomethylformamide whenever obtained by the method referred to in claim 1.

**3.** A method for producing crystalline cefadroxil hemihydrate, which method comprises slurrying at a temperature of from 45 to 55°C a cefadroxil solvate of dimethylacetamide, N-methyl-2-pyrrolidone or monomethylformamide with a mixture of methanol:isopropyl alcohol of from 30:70 to 70:30 by volume containing from 5% to 12% of water and isolating the crystalline cefadroxil hemihydrate thus obtained.

**Patentansprüche**

**1.** Verfahren zur Herstellung eines kristallinen Cefadroxil-hemihydrats, das im wesentlichen die folgenden Röntgen-Beugungseigenschaften aufweist:

| Abstand d (Å) | relative Intensität |
|---|---|
| 10,59 | 30 |
| 8,68 | 100 |
| 8,03 | 43 |
| 7,14 | 39 |
| 6,72 | 41 |
| 6,18 | 40 |
| 5,67 | 11 |
| 5,49 | 23 |
| 4,83 | 69 |
| 4,71 | 52 |
| 4,65 | 25 |
| 4,23 | 57 |
| 4,13 | 55 |
| 3,98 | 74 |
| 3,87 | 33 |
| 3,80 | 36 |
| 3,31 | 30 |
| 3,02 | 15 |
| 2,95 | 18 |
| 2,88 | 25 |
| 2,64 | 25 |
| 2,55 | 19 |
| 2,52 | 17 |
| 2,48 | 14 |
| 2,43 | 15 |
| 2,31 | 16 |
| 2,14 | 13 |
| 2,04 | 9 |

dadurch **gekennzeichnet,** daß zu einer wäßrigen Lösung aus Cefadroxil, welches gerade aus 7-ADCA hergestellt worden ist, ein Lösungsmittel, ausgewählt aus der Gruppe bestehend aus Dimethylacetamid, N-Methyl-2-pyrrolidon, Monomethylformamid, zugegeben wird, während der pH der Lösung innerhalb des Bereiches von 5,5 bis 6 kontrolliert wird, so daß das entsprechende Cefadroxilsolvat erhalten wird, welches ausfällt und abfiltriert wird und das nach dem Trocknen mit einem Gemisch aus Methanol/Isopropylalkohol von 30:70 bis 70:30, ausgedrückt durch das Volumen, welches 5 bis 12% Wasser enthält, bei einer Temperatur im Bereich von +45°C bis +55°C aufgeschlämmt wird, und das so erhaltene kristalline Cefadroxil-hemihydrat aus dem Reaktionsgemisch isoliert wird.

**2.** Cefadroxilsolvate von Dimethylacetamid, von N-Methyl-2-pyrrolidon und von Monomethylformamid, wenn sie nach dem in Anspruch 1 beschriebenen Verfahren erhalten worden sind.

**3.** Verfahren zur Herstellung von kristallinem Cefadroxil-hemihydrat, dadurch **gekennzeichnet,** daß bei einer Temperatur von 45 bis 55°C ein Cefadroxilsolvat von Dimethylacetamid, N-Methyl-2-pyrrolidon oder Monomethylformamid mit einem Gemisch aus Methanol/Isopropylalkohol von 30:70 bis 70:30, ausgedrückt durch das Volumen, welches 5 bis 12% Wasser enthält, aufgeschlämmt wird und das so erhaltene kristalline Cefadroxil-hemihydrat isoliert wird.

## Revendications

**1.** Procédé pour produire du céfadroxil cristallin hémihydraté, présentant essentiellement les propriétés suivantes de diffraction aux rayons X :

| Distance d (Å) | Intensité relative |
|---|---|
| 10,59 | 30 |
| 8,68 | 100 |
| 8,03 | 43 |
| 7,14 | 39 |
| 6,72 | 41 |
| 6,18 | 40 |
| 5,67 | 11 |
| 5,49 | 23 |
| 4,83 | 69 |
| 4,71 | 52 |
| 4,65 | 25 |
| 4,23 | 57 |
| 4,13 | 55 |
| 3,98 | 74 |
| 3,87 | 33 |
| 3,80 | 36 |
| 3,31 | 30 |
| 3,02 | 15 |
| 2,95 | 18 |
| 2,88 | 25 |
| 2,64 | 25 |
| 2,55 | 19 |
| 2,52 | 17 |
| 2,48 | 14 |
| 2,43 | 15 |
| 2,31 | 16 |
| 2,14 | 13 |
| 2,04 | 9 |

dans lequel, à une solution aqueuse de céfradoxil, qui vient d'être préparée à partir de 7-ADCA, on ajoute un solvant choisi parmi l'ensemble comprenant le diméthylacétamide, la N-méthyl-2-pyrrolidone, le monométhylformamide, tout en ajustant le pH de la solution dans l'intervalle de 5,5 à 6, pour obtenir le produit de solvatation correspondant du céfadroxil, qui précipite et est isolé par filtration et qui, après séchage, est mis en suspension dans un mélange 30:70 à 70:30 en volume de méthanol et d'alcool isopropylique, contenant 5 à 12 % d'eau, à une température de +45°C à +55°C, le céfadroxil cristallin hémihydraté ainsi obtenu étant ensuite isolé du mélange réactionnel.

**2.** Produits de solvatation du céfadroxil dans le diméthylacétamide, la N-méthyl-2-pyrrolidone et le monométhylformamide, obtenus par le procédé selon la revendication 1.

**3.** Procédé pour produire du céfadroxil cristallin hémihydraté, lequel procédé consiste à mettre en suspension à une température de 45 à 55°C un produit de solvatation du céfadroxil dans du diméthylacétamide, de la N-méthyl-2-pyrrolidone ou du monométhylformamide, avec un mélange 30:70 à 70:30 en volume de méthanol et d'alcool isopropylique, contenant de 5 à 12 % d'eau, et à isoler le céfadroxil cristallin hémihydraté ainsi obtenu.